# EUROPÄISCHE PATENTSCHRIFT

(11) **EP 1 193 438 B1**
(45) Veröffentlichungstag und Bekanntmachung des Hinweises auf die Patenterteilung: **15.11.2006**
(21) Anmeldenummer: 01119305.9
(22) Anmeldetag: 10.08.2001
(51) Int. Cl.: F16M 11/04, G02B 7/00, G02B 21/00

(54) **Stativ**
Stand
Support

(30) Priorität: 28.09.2000 CH 18982000
(43) Veröffentlichungstag der Anmeldung: 03.04.2002
(73) Patentinhaber: Leica Microsystems Schweiz AG, 9435 Heerbrugg (CH)
(72) Erfinder: Metelski, Andrzej, 8590 Romanshorn (CH)
(74) Vertreter: Rosenich, Paul

(56) Entgegenhaltungen:
- WO-A-97/13997

## Beschreibung

Die Erfindung betrifft ein Stativ mit einer Balanciereinrichtung.

Stative, insbesondere für die Operationsmikroskopie oder dgl. sollten gut ausbalanciert sein, so dass das Mikroskop mit relativ geringer Kraftanstrengung im Raum geschwenkt, gehoben, gesenkt bzw. bewegt werden kann. Der Stand der Technik kennt mehrere Verfahren zum Balancieren und unter anderem auch halbautomatische, bzw. automatische Balancierverfahren, bei denen über einen Kraft- oder Wegsensor ein Balancierstatus gemessen und auf Grund dieser Messung eine elektrisch gesteuerte Balanciermassnahme vorgenommen wird.

Unter elektrisch gesteuerter Balanciermassnahme ist beispielsweise das Ansteuern eines Elektroantriebes zu verstehen, der Balanciergewichte verschiebt. Ein Beispiel einer solchen Balanciereinrichtung ist in der internationalen PCT-Patentanmeldung WO-A-97/13997 der Anmelderin geoffenbart worden.

Die herkömmlichen und bisher veröffentlichten Balanciersysteme mit elektrischer bzw. elektronischer Unterstützung gehen davon aus, dass die Unbalance als physikalische Grösse gemessen wird und auf Grund des Mess-Ergebnisses der Balanciervorgang durchgeführt wird. Dies erfordert entsprechende Kraftmess-Sensoren und einen entsprechenden Messvorgang, auf den erst im Anschluss der Balanciervorgang stattfinden kann. Solche Kraftmess-Sensoren und dazu notwendige elektronische Verstärker sind, da sie sich an bewegten Systemen befinden, in der Regel aufwendig und teuer. Abgesehen davon ist der Balanciervorgang immer reaktiv, weil zuerst gemessen und dann balanciert wird. Bei einigen Stativen aus dem Stand der Technik mit halbautomatischer Balancierung muss, z.B. nach einem Zubehörwechsel, am Stativ ein Knopf gedrückt werden, der den Balanciervorgang auslöst.

Bei solchen Systemen vergeht somit auch einige Zeit, bis der ausbalancierte Zustand erreicht ist.

Die in der Literatur beschriebenen vollautomatischen Balanciersysteme haben gegenüber dem knopfgesteuerten System die - manchmal unerwünschte-Eigenschaft, dass sie ein Stativ auch dann balancieren, wenn der Balanciervorgang nicht erwünscht ist: Beispielsweise, wenn sich ein Chirurg bei einem ausbalancierten Stativ am Mikroskop abstützt oder festhält, kann dies bei vollautomatischen Systemen unverzüglich zu einem Balanciervorgang führen. Dieser Balanciervorgang verursacht beim Nachlassen der Abstützkräfte am Mikroskop, z.B. Wegnehmen der Hände oder Zurücknehmen des Kopfes eine starke Unbalance. Diese muss zuerst wieder detektiert und durch einen neuerlichen Balanciervorgang ausgeglichen werden. Während dieser Zeit ist das Mikroskop für den Chirurgen nicht einwandfrei verwendbar, wodurch er u.U. während dieses Detektions- und Balanciervorganges gehindert ist, die Operation fortzusetzen, bis dieser neuerliche Balanciervorgang abgeschlossen ist. Bei einem vollautomatisch arbeitenden Stativ der Anmelderin (OHS) wird dieses Balancieren daher nicht ständig durchgeführt, sondern nur dann, wenn in den Balanciermodus umgeschaltet wird. Wenn der Assistent jedoch während der Operation Zubehörteile verstellt oder auswechselt, so ist das Mikroskop zunächst nicht mehr ausbalanciert. Um ein einwandfreies Arbeiten zu ermöglichen, ist somit ein erneutes, zeitaufwendiges Balancieren (Umschalten in den Balanciermodus) notwendig, wobei die Operation während dieser Zeit unterbrochen werden muss.

Die Firma Möller-Wedel ist bei ihrem Stativaufbau von einer anderen Überlegung ausgegangen: Zunächst wird eine mechanische Balancierung herbeigeführt. Werden dann Bauteil-Komponenten am Mikroskop ausgetauscht, wird diese Balancierung gar nicht erst bemüht, sondern es werden an den Bauteil-Komponenten selbst, bzw. an anderen am Mikroskop befindlichen Bauteil-Komponenten Einstellungen vorgenommen, die ein neuerliches Balancieren bewirken sollen. Beispielsweise wird der Assistenten-Tubus so verschwenkt, dass ein Balancierzustand erreicht wird.

Dies ist jedoch häufig unbefriedigend, da dadurch eine Position eingestellt wird, die mit der gewünschten Arbeitsposition nicht übereinstimmt.

Der Erfindung liegt die Aufgabe zugrunde, ein neues Balanciersystem zu finden, das auf aufwendige bzw. teure Kraft- oder Wegsensoren verzichtet und einen Balanciervorgang unter Vermeidung einer Unterbrechung der Operation möglichst unmittelbar und rasch ausführt, sobald eine Änderung am Zubehör des Mikroskops auftritt, ohne vorheriges Durchführen eines Messvorganges physikalischer Grössen wie Kräfte, Deformationen oder dgl. und bei dem ein nicht erwünschter Balanciervorgang durch Abstützkräfte - wie oben angegeben - verhindert wird.

Gelöst wird die Erfindung durch die Merkmale des Anspruches 1 bzw. durch die Anwendung der Verfahrensschritte des Anspruches 11.

Das Besondere an der vorliegenden Erfindung ist eine völlig neue Philosophie: Es sollen nicht physikalische Kräfte, bzw. Wege (Deformationen) gemessen werden, die etwas über den Balancezustand aussagen, sondern es soll ein vollständiges mathematisches rechnergestütztes Modell des Stativs vorgesehen werden. Dabei wird durch Hinzufügen oder Wegnehmen von Komponenten zu diesem Modell (Hinzufügen oder Wegnehmen von Zubehör zum Mikroskop oder Lageverändern von wesentlichen Bauteilen) das Berechnungsmodell entsprechend geändert, bzw. die entsprechende Änderung des Modells rechnerisch berücksichtigt und auf Grund des rechnerischen Modells die Steuerbefehle für die elektrische Ansteuerung von Ausgleichsgewichten ausgelöst.

Im Vergleich zu den bisherigen mehr oder weniger intelligenten Balanciersystemen (Unbalance wird mittels Intelligenz gemessen und mittels intelligenter Steuerung ausbalanciert) kommt erfindungsgemäss erstmals ein intelligentes Stativ zum Einsatz. (Das Stativ kennt seinen Aufbau durch intelligenten Rechnereinsatz und entsprechende Programmierung bzw. Eichung und erkennt jede Änderung an seinem Aufbau, um dementsprechende Balanciermassnahmen mittels direkter Ansteuerung von Ausgleichsgewichten o.dgl. vorzunehmen.)

Erfindungsgemäss ist somit zunächst ein Rechner vorgesehen, der ein Programm beinhaltet, mit dem die Balancezustände gerechnet werden können und die Nichtbalance rechnerisch ermittelt wird, indem die hinzugefügten oder entfernten Komponenten (z.B. Zubehörteile am Mikroskop) mit ihren das Gesamtmodell beeinflussenden Eigenschaften eingegeben werden. Diese Eingabe findet vorzugsweise in codifizierter Form statt, so dass beispielsweise manuell oder mittels Barcodeleser oder dgl. Informationen über das hinzugefügte oder entfernte Zubehörteil zum Mikroskop dem Rechner eingegeben werden.

So trägt beispielsweise jedes Zubehörteil eine Nummer, die der Anwender dem Rechner über eine Tastatur mitteilt, bevor er das Zubehörteil montiert. Um diesen Vorgang zu beschleunigen ist - entsprechend einer besonderen Ausbildung der Erfindung - ein automatisches Erkennen der Zubehörteile vorgesehen. Dies erfolgt beispielsweise durch einen automatisch ablesbaren Barcode, der an einer Stelle des Zubehörteiles angebracht ist, die beim Montieren automatisch von einem Lesegerät erfasst wird.

Gemäss einer weiteren besonderen Ausbildung der Erfindung ist an Stelle eines Barcodes ein Mikrochip mit den entsprechenden eingeschriebenen Informationen vorgesehen. (Beispielsweise ein Mikrochip wie auf einer Scheckkarte).

Selbstverständlich können auch magnetische oder andere Datenträger oder dgl. für die entsprechende Informationsübertragung zwischen Zubehörteil und Rechner sorgen. Günstig ist bei diesem Aufbau, dass der Rechner ohne weiteres Zutun automatisch und unmittelbar erkennt, welche Zubehörteile vom Mikroskop entfernt wurden und welche hinzugefügt wurden. Da alle diese Bauteile im rechnerischen Modell erfasst sind, kann unmittelbar und unverzüglich die richtige Balancierposition der Ausgleichsgewichte eingestellt werden.

Das Balancieren erfolgt somit wesentlich schneller als bei herkömmlichen Stativen. Die Intelligenz für den eigentlichen Balanciervorgang kann dabei sogar reduziert (und dadurch billiger) als bei den bekannten halb- oder vollautomatischen Balanciersystemen sein.

Ein einmal durchgeführter Balanciervorgang wird bei einem erfindungsgemässen Aufbau auch nicht durch ein Aufstützen des Kopfes oder durch die Hände beeinflusst. Solche Vorgänge werden durch den Rechner bzw. das mathematische Modell gar nicht erst erfasst.

Eine weitere besondere Ausgestaltung der Erfindung sieht vor, dass zusätzlich oder alternativ zum informationsübertragenden Teil (Chip, Barcode oder dgl.) Winkel- oder Weg-Sensoren vorgesehen sind, welche die Stellungen von schwenkbaren Teilen des Zubehörs erfassen und auf Grund einer bestimmten Schwenklage dieser Teile dem Rechner (gegebenenfalls über den Chip) bzw. dem Berechnungsmodell des Balanciersystems eine Veränderung signalisieren, die eine Balancierkorrektur ermöglicht bzw. triggert. Im Unterschied zu den bisher bei Balanciersystemen eingesetzten Kraftmess-Sensorsystemen, sind Winkelsensoren sehr preisgünstig und es werden an sie auch keine besonders hohen Anforderungen gestellt, da geringfügige Änderungen der Winkellage, z.B. eines Tubus, sich nicht wesentlich auf die Unbalance eines ausbalancierten Stativs auswirken.

Im Zuge einer Weiterentwicklung dieser erfinderischen Ausbildung ist für den Winkelsensor bzw. für den mit ihm verbundenen Chip eine geeignete Logik vorgesehen, die permanent die entsprechende Balancierinformation an den Rechner liefert. In diesem Fall ist eine Codifizierung und eine tabellarische Ablage der Zubehörbauteil-Informationen im Rechner nicht unbedingt erforderlich, sondern das mathematische Modell berechnet sich jeweils automatisch auf Grund der durch das jeweilige Zubehör selbsttätig eingelesenen Balancier-Informationen.

Für die Realisierung der obigen erfindungsgemässen Massnahmen, ist der konkrete Aufbau des Stativs nicht wesentlich. Es kann sich dabei um Aufbauten mit Parallelogrammträgern oder auch um Aufbauten mit normalen Balkenträgern oder dgl. handeln. Herkömmliche Stative können mit der erfindungsgemässen Lösung problemlos nachgerüstet werden, da in der Regel lediglich zusätzliche Datenleitungen und Informations-Abgreifelemente montiert werden müssen.

Alternativ zu Datenleitungen und elektronisch verbundenen Systemen sind auch Zubehörteile denkbar, die mittels Infrarot-Sensoren oder anderen Fernübertragungs-Einrichtungen (wie z.B. Ultraschall oder Funkwellen) mit dem Rechner verbindbar sind. So können beispielsweise Infrarot-Leuchtdioden oder Reflektoren, wie beispielsweise in der Europäischen Patentanmeldung EP-A-822 436 der Anmelderin angegeben, aufgebaut sein. D.h. Lichtimpulse, die vom Zubehörteil ausgehen, signalisieren dem Rechner über entsprechende Lichtimpulsempfänger ihre Balanciereigenschafts-Informationen.

Durch die Erfindung wird es auch möglich, einen Standard für Zubehörteile zu entwickeln, der ein universelles Verwenden von Zubehörteilen an verschiedenen Systemen mit einem erfindungsgemässen Balancierrechner erlaubt. Die Erfindung erlaubt es auch, kundeneigene Zubehörteile im Betrieb des Herstellers zu eichen, indem dort ein Referenzstativ vorgesehen ist, bei dem über herkömmliche Balanciersystem-Messeinrichtungen die Balanciereigenschaft des entsprechenden Zubehörteiles bestimmt und anschliessend codifiziert an diesem festgelegt wird. Dieses kann z.B. über den Barcode, einen Chip oder eine andere Codierung, die gegebenenfalls mittels Computerdiskette mitgeliefert wird, erfolgen.

Selbstverständlich ist die Erfindung nicht auf eine reine Balancierausrüstung entsprechend den obigen Erfindungsmassnahmen eingeschränkt, sondern sie gestattet auch eine Kombination mit herkömmlichen automatischen oder halbautomatischen Balanciersystemen, sofern für den Anwender daraus Vorteile ableitbar sind. Entscheidend ist jedenfalls, dass durch das erfindungsgemässe Balanciersystem direkt und unmittelbar, ohne weitere Messvorgänge, eine optimale und unverzügliche Balancierung bewirkt werden kann.

Besonders geeignet ist die vorliegende Erfindung auch für das Balancieren direkt am Optikträger, d.h. für Schwenkbewegungen des Mikroskops um eine horizontale Achse und um wenigstens eine weitere Achse, die in einem Winkel zur horizontalen Achse angeordnet ist. Für das erfindungsgemässe Balancieren bei einem Schwenkträger (ein Schwenkträger ist jenes Bauteil, welches das Mikroskop unmittelbar hält und welches dem Mikroskop über wenigstens eine, in der Regel zwei bis drei, Schwenkachsen, eine Beweglichkeit im Raum gibt und welches selbst an Tragteilen des Stativs befestigt ist) wird erfindungsgemäss die Balancierung so vorgenommen, dass über die elektrischen Antriebe die Schwerpunktachsen und der Schnittpunkt der Schwenkachsen des Stativs relativ so zueinander verschoben werden, dass der Schnittpunkt wenigstens zweier Schwenkachsen (es können auch drei oder mehr Schwenkachsen sein) im Schwerpunkt des Mikroskops bzw. des mit dem Mikroskop unmittelbar verbundenen Aufbaus zu liegen kommt.

Diese Balanciermassnahme kann bewerkstelligt werden durch das Verschieben von Ausgleichsgewichten, die den Schwerpunkt des Mikroskops bzw. des Aufbaus verschieben; oder durch das Verschieben der Schwenkachsen, so dass deren Schnittpunkt im Schwerpunkt zu liegen kommt, oder durch das Verschieben des Mikroskops bzw. der betroffenen Zubehörteile relativ zu den entsprechenden Schwenkachsen des Mikroskops, so dass sein Schwerpunkt auf den Schnittpunkt der Achsen gelegt wird.

Eine optimale Ausbalancierung am Schwenkträger ist dann gegeben, wenn der Schnittpunkt aller Schwenkachsen exakt im Schwerpunkt des bewegbaren Aufbaus (Mikroskop mit Zubehör) liegt.

Die Erfindung sieht bei einer Ausbildung insbesondere vor, dass an einem Stativ sowohl im Schwenkträger als auch an den übrigen Trägern erfindungsgemäss balanciert wird.

Die Erfindung wird nachstehend, anhand der sie beispielsweise und symbolisch darstellenden Ausführungsbeispielen näher erläutert.

Es zeigen:
- Fig. 1: ein x/y/z-Koordinatensystem in dem die Schnittpunkte der Schwenkachsen und der Schwerpunktachsen zueinander verschoben werden,
- Fig. 2 bis 4: herkömmliche Operationsmikroskopie-Aufbauten mit einem Schwenkträger und neuen Balancierantrieben,
- Fig. 5: einen herkömmlichen Stativaufbau, mit innenliegenden codifizierten Informationen an den Schnittstellen,
- Fig. 6: einen Aufbau entsprechend der Fig. 11 der oben erwähnten WO-A-97/13997, jedoch mit einem Code-Lesegerät und einem Rechner,
- Fig. 7: einen symbolischen Stativaufbau mit Elektromotoren (Stellmotoren), welche die Ausgleichsgewichte rechnergestützt verschieben,
- Fig. 8: einen Stativbalken mit Rechner und zwei Ausgleichsgewichten,
- Fig. 9: ein Detail des Aufbaues gemäss Fig. 6,
- Fig. 10: eine Symboldarstellung eines Zubehöraufbaues mit codifizierten Datenträgern,
- Fig. 11: eine Vielzahl von beispielhaften Zubehörteilen, von denen jedes codifiziert ist,
- Fig.12 und 13: Beispiele von unterschiedlichen Varianten von Datenträgern,
- Fig. 14: ein Zubehörteil mit Winkelsensor und Barcode,
- Fig. 15: ein Modell des Gesamtaufbaus,
- Fig. 16: symbolisch einen Mikroskopaufbau mit drei Bauteilen und geometrisch angeordneten Signalgebern.

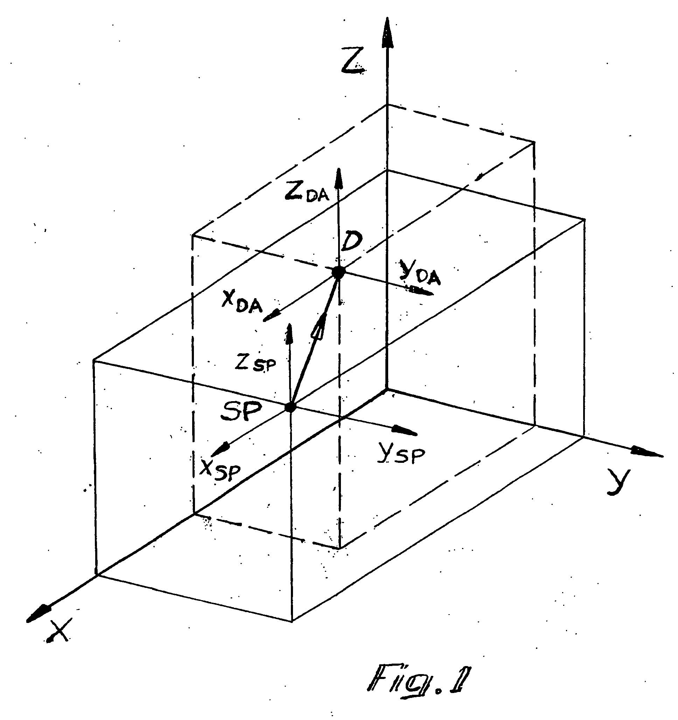

Aus Fig. 1 ist das rechnergestützte Zueinanderverschieben des Schnittpunktes D der Schwenkachsen x_{DA}/y_{DA} und z_{DA} und des Schnittpunktes SP der Schwerpunktachsen x_{SP}/y_{SP} und z_{SP} in einem x/y/z-Koordinatensystem eines Schwenkträgers ersichtlich. Dieses Zueinanderverschieben ist erforderlich, damit das System ausbalanciert ist, mit geringem Kraftaufwand verstellt werden kann und in jeder beliebigen Operationslage im Gleichgewicht ist.

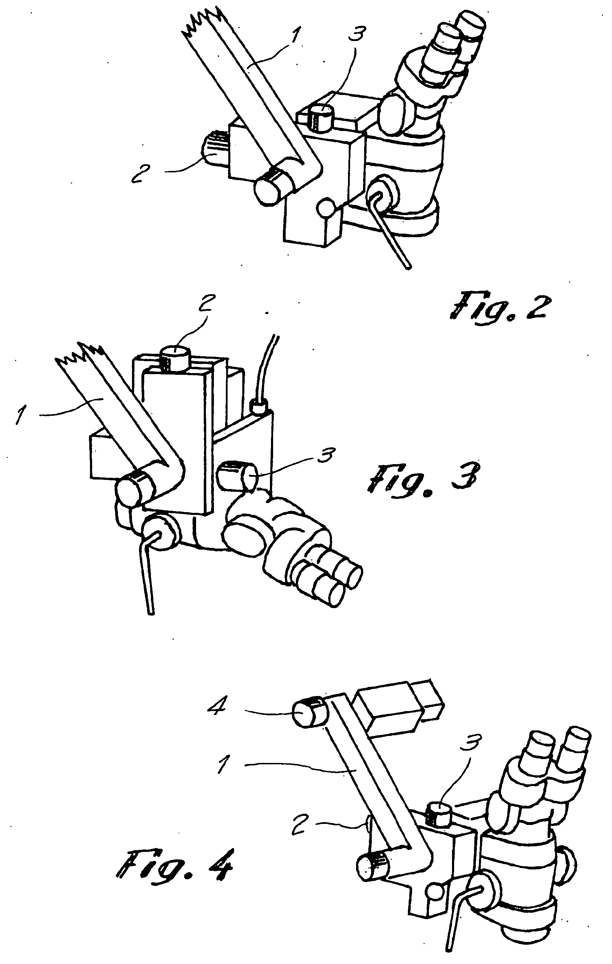

Die Fig. 2 bis 4 zeigen modifizierte herkömmliche Operationsmikroskopie-Aufbauten mit einem Schwenkträger 1 und einem automatischen Balanciersystem, das erfindungsgemäss angesteuert ist. Anstelle der bisher verwendeten Handschrauben zur Balancierung befinden sich durch den Rechner angesteuerte Elektromotoren 2, 3 und 4 am Mikroskop. Der Rechner erhält seine Informationen durch die Codifizierungs-Bauteile, die in den Figuren 2-4 nicht sichtbar sind, da sie sich jeweils im Geräteanschlussbereich (Schnittstelle) befinden.

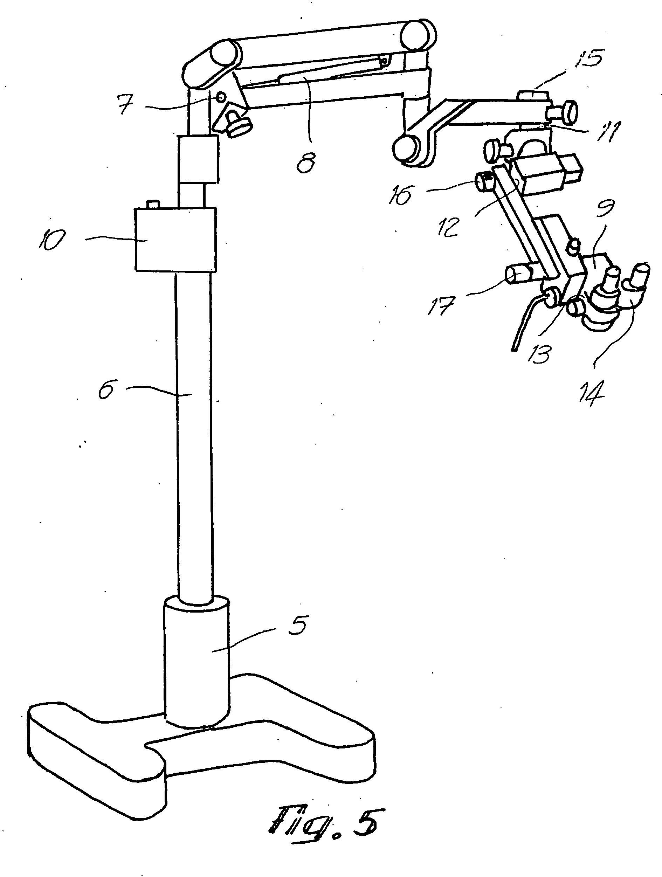

Bei dem aus Fig. 5 ersichtlichen, herkömmlichen Stativaufbau mit einem Ständerfuss 5 und einer Tragsäule 6 wird rechnergesteuert der eine Abstützpunkt 7 einer Stützfeder 8 in Abhängigkeit von Gewichtsänderungen im Bereich des Mikroskops 9 verschoben. Die Gewichtsänderungen durch Auswechseln oder Verstellen der Zubehörteile erfährt ein an der Tragsäule 6 angeordneter Rechner 10 durch die codifizierten Informationen, die von den jeweilig hinzugefügten Zubehörteilen (z.B. Schwenktubus) von den Schnittstellen 11, 12, 13 und 14 automatisch an den Rechner 10 weitergeleitet werden. Zusätzliche Informationen erhält der Rechner 10 von Winkelsensoren 15, 16 und 17, welche im Bereich der Gelenke angeordnet sind und die jeweilige Stellung des Zubehörs detektieren und an den Rechner 10 weitergeben.

Die in der Fig. 5 angegebenen Winkelsensoren werden bei einem Aufbau mit Stützfeder 8 und Parallelogrammträgern in der Regel nicht benötigt, da die Lageänderung der Bauteile am Ende des Parallelogrammträgers zu keiner Balanceänderung führt. Wird jedoch die Stützfeder 8 versteift, bzw. fixiert oder der Parallelogrammträger durch einen einfachen Horizontalbalken ersetzt, so führt die Winkeländerung um die angegebenen Winkelsensoren auch zu Änderungen des Balancezustandes. Andererseits führen bei jeder Lösung die angegebenen Winkeländerungen zu einer Änderung der Kippsicherheit des Stativs über den Stativfuss 5. Insofern können solche Winkelsensoren auch vorgesehen sein, um Informationen hinsichtlich der Kippsicherheit zu ermitteln und in das Balanciersystem einzugeben, das beispielsweise mittels Alarmsignal vor einem bevorstehenden Kippen des Stativs warnt.

Ein solches Kippwarnsystem stellt eine eigene unabhängige Erfindung dar, da es bei beliebigen Operationsmikroskop-Stativen sinnvoll zur Anwendung gelangen kann.

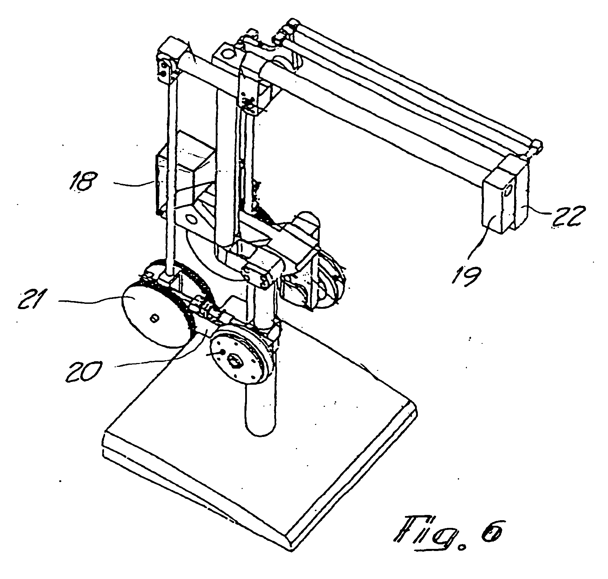

Der Aufbau gemäss Fig. 6 entspricht demjenigen der Fig. 11 der erwähnten WO-A-97/13997. Im Unterschied zum Aufbau gemäss der WO-A-97/13997 ist ein Elektromotor 20 zur Verstellung des Ausgleichsgewichtes 21 nicht durch einen Gleichgewichts-Sensor, sondern durch einen Rechner 18 gesteuert, wobei der Rechner 18 seine Informationen über ein Lesegerät 19 in codifizierter Form von den Zubehörteilen erhält, die vorne unterhalb des Bauteiles 22 montiert werden können.

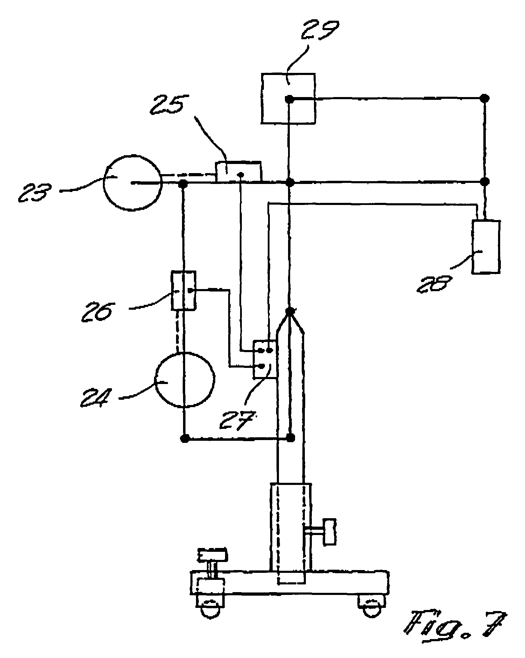

Bei der aus Fig. 7 ersichtlichen, symbolischen Darstellung werden Ausgleichsgewichte 23, 24 rechnergestützt mittels Elektromotoren (Stellmotoren) 25, 26 verschoben. Die Informationen für den Rechner 27 kommen erfindungsgemäss direkt von der Last 28 und/oder von einem Winkelsensor 29 über elektrische Verbindungsleitungen.

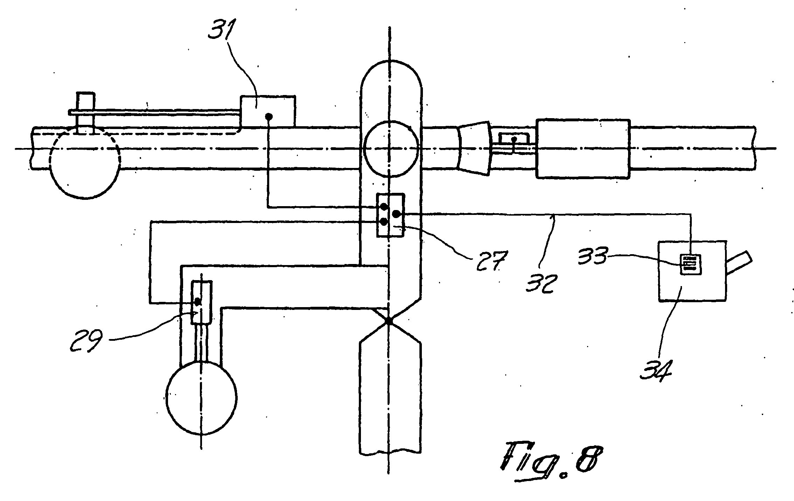

Der in Fig. 8 gezeigte Stativaufbau weist einen Rechner 27, Verstellmotoren 31, eine Steuerleitung 32 und symbolisch einen Chip 33 am Mikroskop 34 auf, der die relevanten Informationen für den Rechner 27 enthält. Ebenso ist ein Winkelsensor 29 angedeutet, der Schwenkbewegungen um eine Vertikalebene erfasst und an den Rechner 27 weitergibt.

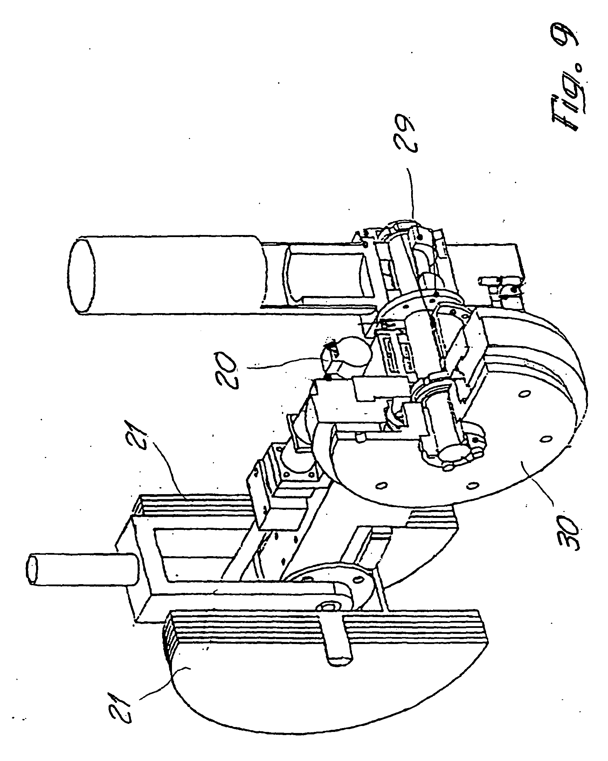

Aus der Fig. 9 ist ein Detail des Aufbaues gemäss Fig. 6 in vergrössertem Massstab ersichtlich. Dabei ist der Elektromotor 20 zum Verstellen der Ausgleichsgewichte 21 deutlich ersichtlich. Ebenso erkennt man eine herkömmliche Bremse 30 zur Fixierung einer Position und einen Winkelsensor 29, der die Schwenklage angibt.

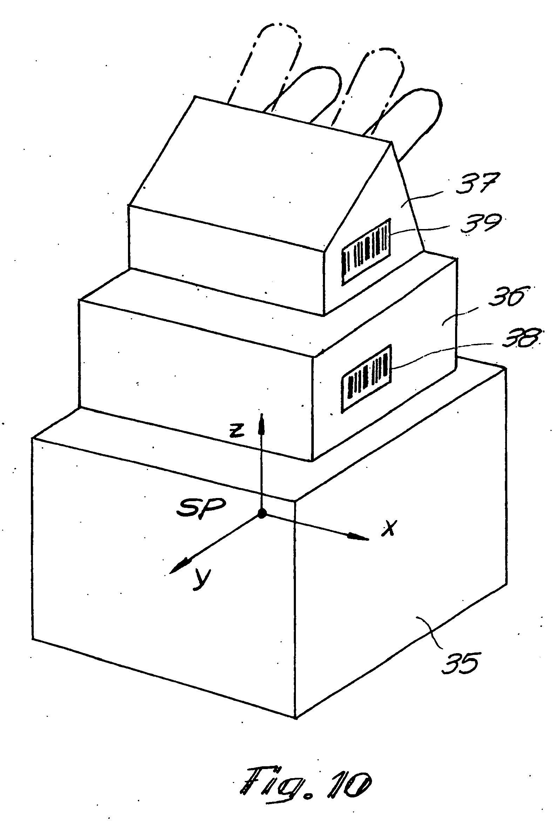

Die in Fig. 10 dargestellte Anordnung zeigt symbolisch einen Aufbau von Zubehörteilen. Dabei sind Zubehörteile 36 und 37 mit einem Bauteil 35 lösbar verbunden. Durch den Anbau der Zubehörteile 36, 37 verlagert sich der Gesamtschwerpunkt SP des Systems. Die Zubehörteile 36, 37 sind mit Datenträgern 38, 39 versehen, von welchen die erforderlichen Korrekturdaten (können auch Buchstaben oder Zahlencodes sein) von einem Lesegerät automatisch oder von Hand eingelesen werden und an den Rechner weitergegeben werden können.

Aus Fig. 11 ist eine Vielzahl von beispielhaften Zubehörteilen ersichtlich, welche zu einem Zubehörsystem gehören. Jedes Zubehörteil ist für sich codifiziert und zum Einsatz einzeln oder in Kombination mit anderen Zubehörteilen an einem Operationsmikroskop verwendbar.

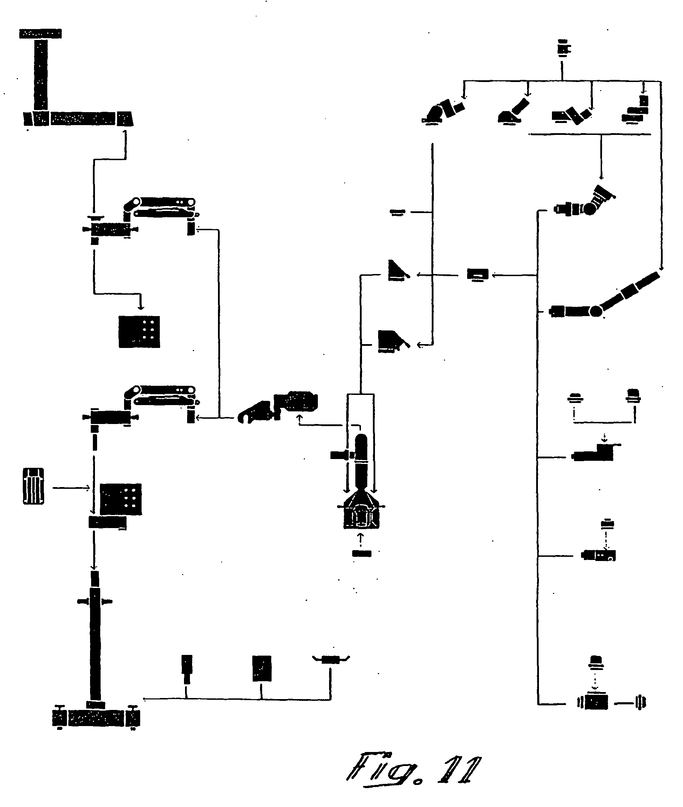

Die Fig.12 zeigt einen mit sogenanntem Strich- oder Barcode versehenen Datenträger 40 für die an den Zubehörteilen anzubringenden Korrekturdaten für die Balanciersteuerung. Dieser Datenträger 40 ist beispielsweise als Selbstklebe-Etikett ausgebildet.

Aus Fig. 13 ist ein weiterer Datenträger 41 mit einem Mikrochip, wie er beispielsweise auf Kreditkarten verwendet wird, ersichtlich. Solche Mikrochips oder auch Magnetstreifen, wie sie beispielsweise ebenfalls auf Kreditkarten verwendet werden, können vom Hersteller des Zubehörteils oder gegebenenfalls auch vom Anwender auch nachträglich programmiert werden.

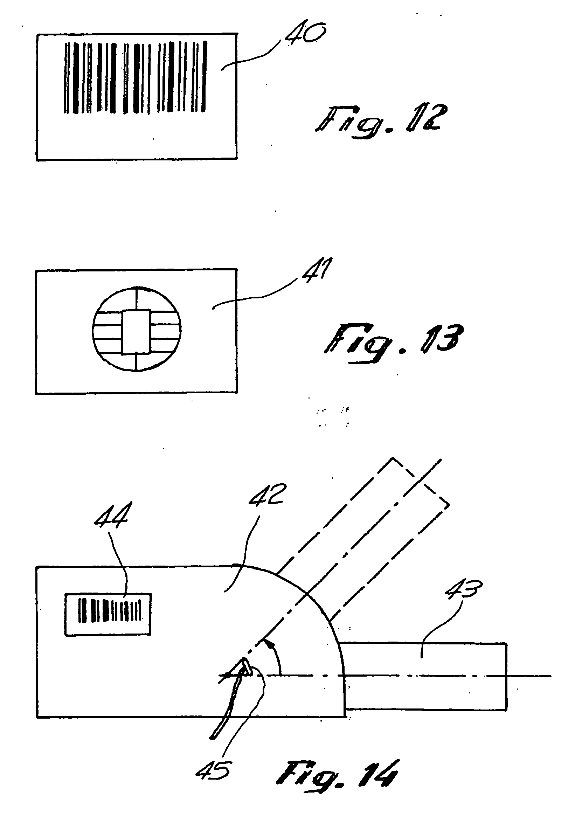

Das in Fig. 14 symbolisch dargestellte Zubehörteil 42 weist beispielsweise einen sogenannten Schwenktubus 43, einen Barcode-Datenträger 44 sowie einen Winkelsensor 45 auf. Der Winkelsensor 45 dient zur Erfassung der jeweiligen Position des verstellbaren Schwenktubus 43, welche die Balance des Systems ebenfalls beeinflussen kann.

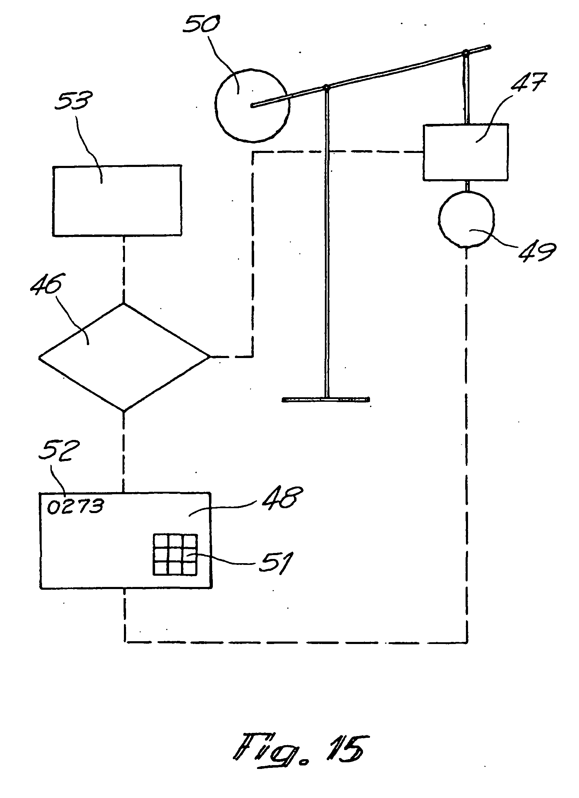

Das aus Fig. 15 ersichtliche schematische Modell eines Gesamtaufbaus zeigt ein Informationssystem 46, das die an den Zubehörteilen 47 gespeicherten fixen Daten sowie allfällige, von der Arbeitsstellung der Zubehörteile 47 abhängige Daten an einen zentralen Rechner 48 weiterleitet. Dieser Rechner 48 ermittelt die erforderlichen Korrekturwerte und gibt diese als Sollwerte an das Balanciersystem 49, 50 weiter. Das Balanciersystem kann dabei auch bloss das eigene Gewicht, d.h. das Mikroskop oder das entsprechende Bauteil ortsverschieben, um die Balancierwirkung zu erzielen. Die Korrekturdaten können vom Zubehörteil 47 auch von Auge abgelesen und manuell über eine Tastatur 51 in den Rechner 48 eingegeben werden. Zur Kontrolle der eingegebenen Werte kann beispielsweise eine digitale Anzeige 52 dienen.

Das System kann zusätzlich mit einer intelligenten Komponente 53, (beispielsweise Mikrochip), versehen werden, welche Abfragen vornehmen oder beispielsweise zum Eichen von neuen Zubehörteilen verwendet werden kann.

Alternativ zum angegebenen Verschieben von Ausgleichsgewichten in den Balanciersystemen 49 oder 50, ist es erfindungsgemäss vorgesehen, das gesamte auszubalancierende System über die entsprechende Balanceachse zu schieben, bzw. den Schwerpunkt in diese entsprechenden Achsen zu legen. So ist beispielsweise vorgesehen, am senkrechten Ständer unterhalb der Lagerstelle für den Querbalken eine X/Y-Verstelleinheit vorzusehen, welche die Relativlage des Querbalkens zum Ständer ändert, sobald das Gewicht beim Balanciersystem 49 durch Hinzugeben von zusätzlichen Bauteilen oder Wegnehmen solcher Bauteile geändert wird. Dies ist lediglich eine beispielhafte Angabe, die das Prinzip verdeutlichen soll.

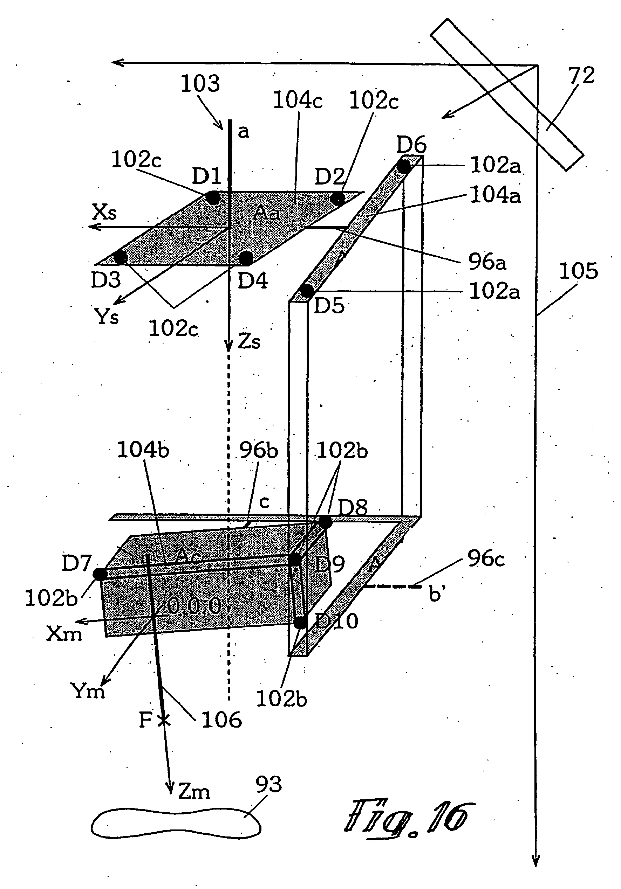

Die Fig. 16 zeigt symbolisch ein Mikroskop, wie es an einer Achse 96c an einem Stativ befestigt sein kann. Das Mikroskop umfasst drei voneinander unabhängige, jedoch miteinander verbundene Bauteile 104a, 104b und 104c. Jedes dieser Bauteile 104 ist mit Signalsendern 102 versehen, die eine doppelte Funktion haben können. Bei der einen Funktion können sie in codierter Form Signale aussenden, die Auskunft über die Eigenschaft des entsprechenden Bauteils geben. Solche Signale können durch den Empfänger 72 empfangen werden und können somit zur Identifizierung der hinzugefügten oder weggenommenen Bauteile verwendet werden. Als Signalsender kommen beispielsweise in Frage: Leuchtdioden, Ultraschallsender, Funksender oder Lichtreflektoren, die durch Fremdlicht angeregt und mit einer entsprechenden Reflexionsinformation die Identifizierung ermöglichen. Als zweite Funktion im Rahmen der Erfindung liegende Variante können diese Sensoren dank ihrer geometrischen Anordnung auch zur Identifizierung von bestimmten Positionen und Lagen der entsprechenden Bauteile beitragen. Diese Positionen und Lagen werden ebenso durch den Empfänger 72 registriert und über den Rechner zu den entsprechenden Balancier-Informationen umgewandelt.

In einem weiteren unabhängigen Erfindungsschritt kann bei einem solchen Aufbau, bei dem die Erfassung der Bauteile, deren Eigenschaften und deren Lage vollständig von aussen erfolgt, in umgekehrter Weise eine Steuerung der Balancierantriebe ebenfalls von aussen erfolgen. Die diesbezügliche Erfindung sieht vor, dass die durch den Empfänger 72 empfangenen Signale nach Umwandlung im Rechner zu Steuersignalen verarbeitet werden, die über eine vergleichbare Sendeanlage wieder auf das Mikroskop, bzw. das Stativ zurückgesendet werden, um dort die entsprechenden Balancier-Stellantriebe anzusteuern. In der gegenständlichen Fig. 16 kann man sich beispielsweise vorstellen, dass eine Verschwenkung des Bauteils 104a um die Achse 96c zu einer Unbalance führt, die durch eine Verschwenkung des Bauteiles 104c um die Achse 96a ausgeglichen wird.

In keinem Fall wird dabei eine Unbalance gemessen. In diesem Beispiel wird vielmehr erfindungsgemäss durch den Empfänger 72 die Lageveränderung der Dioden D5 und D6 erkannt, die Unbalance berechnet und die entsprechenden Steuersignale an einen nicht dargestellten Stellmotor zum Verstellen des Teiles 104c im Raum, z.B. um die Achse 96a oder mit der Achse 96a weitergegeben.

Der Stellmotor ist entweder ein Schrittmotor, der an eine vorgegebene Position fährt oder ein beliebiger Motor, der das Bauteil 104c solange verstellt, bis der Empfänger aufgrund der geänderten Position der Dioden D1, D2, D3 und D4 die rechnerisch ermittelte richtige Balancierlage erkennt und den Verstellvorgang beendet. Das Bauteil 104b ist entsprechend mit Dioden D7, D8, D9 und D10 versehen.

In der Fig. 16 sind zur Veranschaulichung noch die entsprechenden Koordinatensysteme 105, 106 und 103 dargestellt, ebenso wie symbolisch ein Objekt 93.

### Bezugszeichenliste

- 1: Schwenkträger
- 2: Elektromotor
- 3: Elektromotor
- 4: Elektromotor
- 5: Ständerfuss
- 6: Tragsäule
- 7: Abstützpunkt
- 8: Stützfeder
- 9: Mikroskop
- 10: Rechner
- 11: Schnittstelle
- 12: Schnittstelle
- 13: Schnittstelle
- 14: Schnittstelle
- 15: Winkelsensor
- 16: Winkelsensor
- 17: Winkelsensor
- 18: Rechner
- 19: Lesegerät
- 20: Elektromotor
- 21: Ausgleichsgewicht
- 22: Bauteil
- 23: Ausgleichsgewicht
- 24: Ausgleichsgewicht
- 25: Elektromotor
- 26: Elektromotor
- 27: Rechner
- 28: Last
- 29: Winkelsensor
- 30: Bremse
- 31: Verstellmotor
- 32: Steuerleitung
- 33: Chip
- 34: Mikroskop
- 35: Bauteil
- 36: Zubehörteil
- 37: Zubehörteil
- 38: Datenträger
- 39: Datenträger
- 40: Barcode-Datenträger
- 41: Microchip-Datenträger
- 42: Zubehörteil
- 43: Schwenktubus
- 44: Barcode-Datenträger
- 45: Winkelsensor
- 46: Informationssystem
- 47: Zubehörteil
- 48: Rechner
- 49: Balancier-System
- 50: Balancier-System
- 51: Tastatur
- 52: Anzeige
- 53: Intelligente Komponente

- 72: Signal-Empfänger und/oder Sender
- 96a, 96b, 96c: Achsen
- 102a, 102b, 102c: Signalgeber
- 104a, 104b, 104c: Bauteil
- D1 bis D10: Dioden (Signal-Sender und oder Empfänger)

## Patentansprüche

1. Stativ mit einem automatisch ausregulierbaren Balanciersystem mit elektrisch gesteuerten Balanciermechanismen und einem Rechner (10, 18, 27,48)mit einem ihm zugeordneten Berechnungsprogramm zur Ansteuerung der Balanciermechanismen, mit wenigstens einem, mit dem Stativ beweglich verbundenen Bauteil (35) und mit dem Bauteil (35) lösbar verbindbarem, auswechselbarem Zubehör (36, 37), **dadurch gekennzeichnet, dass** dem Rechner (10, 18, 27, 48) ein Informationssystem (46) zugeordnet ist und das Zubehör (36, 37) wenigstens einen durch das Informationssystem (46) mittels Lesegerät (19) ablesbaren Datenträger (38, 39, 40, 41) für Balance-Korrekturdaten des Zubehörs (36, 37) aufweist, oder dass das Zubehör (36, 37) wenigstens einen ablesbaren Datenträger (38, 39) für Balance-Korrekturdaten des Zubehörs (36. 37) aufweist, der durch eine Bedienungsperson ablesbar und dessen Daten in das Informationssystem eingebbar sind.

2. Stativ nach Anspruch 1 **dadurch gekennzeichnet, dass** der Rechner (27) ein Berechnungsprogramm umfasst, das - gegebenenfalls in Abhängigkeit von Winkel- oder Verschiebestellungen zueinander beweglicher Teile von Zubehör (36) oder Zubehörteilen (37) - eine Relativbewegung zwischen den Schwenkachsen (XDA/YDA/ZDA) und den Schwerpunktachsen (XSP/YSP/ZSP) des betroffenen Bauteils so steuert, dass der Schnittpunkt (D) der Schwenkachsen (XDA/YDA/ZDA) im Gesamtschwerpunkt (SP) des beweglichen Bauteils liegt.

3. Stativ nach Anspruch 1 oder 2, **dadurch gekennzeichnet, dass** das Informationssystem (46) eine Dateneingabeeinheit für auf dem Datenträger des Zubehörs (47) enthaltene Daten aufweist.

4. Stativ nach Anspruch 3, **dadurch gekennzeichnet, dass** die Dateneingabeeinheit zumindest eine manuell betätigbare Tastatur (51) und eine Anzeige (52) aufweist.

5. Stativ nach Anspruch 3, **dadurch gekennzeichnet, dass** die Dateneingabeeinheit ein automatisches Lesegerät (19) für die auf dem Datenträger (38, 29) des Zubehörteils (36, 37) gespeicherten Daten umfasst.

6. Stativ nach einem der vorhergehenden Ansprüche, **dadurch gekennzeichnet, dass** bewegliche Bauteile, verschieb- oder schwenkbares Zubehör (42) oder darauf verschieb- oder verschwenkbare Zubehörteile (43) Weg- und/oder Winkelmesssysteme aufweisen, welche bei Lageveränderungen entsprechende, zusätzliche Korrekturdaten zur Berücksichtigung beim Balanciervorgang an den Rechner (48) weiterleiten.

7. Stativ nach einem der vorhergehenden Ansprüche, **dadurch gekennzeichnet, dass** das Informationssystem einen Signalempfänger (72) umfasst, der aus der Entfernung Signale aufnehmen kann, die von Signalsendern (D1-D10) an den Zubehörteilen (102-104) abgesandt werden.

8. Stativ nach Anspruch 7, **dadurch gekennzeichnet, dass** die Signalsender (D1-D10) frequenz- oder farbcodierte Leuchtdioden, Ultraschallsender oder durch Fremdlicht erregbare, diskriminierbare Reflektorelemente sind.

9. Stativ nach einem der vorhergehenden Ansprüche, **dadurch gekennzeichnet, dass** das Informationssystem (46) FemUbertragungseinrichtungen, wie beispielsweise Infrarot-, Ultraschall- oder Funksender/-empfänger umfasst.

10. Stativ nach einem der vorhergehenden Ansprüche, **dadurch gekennzeichnet, dass** der Datenträger (41) wenigstens einen intelligenten Chip umfasst, der im Betriebszustand - gegebenenfalls über am Zubehör (47) angeordnete Sensoren (45) - selbsttätig balancerelevante Daten ermittelt und an das Informationssystem (46) liefert.

11. Verfahren zum automatischen Ausbalancieren von regulierbaren Balanciersystemen an einem Stativ mittels eines Rechners (48), mit einem oder mehreren mit dem Stativ beweglich verbundenen Bauteilen (22, 35) und damit an einer Befestigungsbasis lösbar verbindbarem, auswechselbarem Zubehör (36, 37, 47), **dadurch gekennzeichnet, dass**
a) das Gewicht und die Schwerpunktslage des montierten Zubehörs (36, 37, 47) gegenüber der Befestigungsbasis (35) mittels Informationssystem (46) digital erfasst und zur Korrektur an den Rechner (48) weitergeleitet werden, **oder dass**
b) das Gewicht und die Schwerpunktslage des Zubehörs (36, 37, 47) an der Befestigungsstelle an einem Datenträger (37, 38, 40, 41, 44) angegeben sind und dass eine Bedienungsperson diese Daten zur Balance-Korrektur des Stativs in ein Informationssystem (46) eingibt, das mit dem Rechner (48) verbunden ist, **und dass**
in beiden Fällen a) und b) anschließend der Rechner (48) elektrische Stellmotoren zur Balancierung direkt ansteuert.

12. Verfahren nach Anspruch 11, **dadurch gekennzeichnet, dass** die jeweiligen Verschiebe- oder Winkelstellungen beweglichen Zubehörs (37, 38, 40, 41, 44) oder beweglicher Zubehörteile (43) erfasst und über das Informationssystem (46) selbsttätig zur zusätzlichen Korrektur des Balanciervorgangs in den Rechner (48) eingegeben werden.

13. Verfahren nach Anspruch 12, **dadurch gekennzeichnet, dass** dem Zubehör ein Chip (33) mit eigener Logik zugeordnet ist, der selbsttätig die Verschiebe- und Winkelstellungen ermittelt und deren für den Rechner programmrelevanten Parameter ermittelt und diese über das Informationssystem (46) direkt in den Rechner (48) einspeist, sodass dieser bezüglich solcher Lage- oder Winkeländerungen erzeugten Unbalancezuständen keine weiteren zusätzlichen Berechnungen durchführen muss, sondern alle vom Informationssystem (46) gelieferten Daten als Normdaten übernimmt.

14. Verfahren nach einem der Ansprüche 11 bis 13, **dadurch gekennzeichnet, dass** ein lokales Schwerpunkt-Koordinatensystem definiert und der tatsächliche Schwerpunkt (SP) des beweglichen Bauteils (35) in diesem Koordinatensystem berechnet wird und rechnerisch die Relativlage von wenigstens zwei Schwenkachsen des beweglichen Bauteils (35) ermittelt werden und die elektrischen Stellmotoren (2, 3, 4) so angesteuert werden, dass der Schnittpunkt (D) dieser wenigstens zwei Schwenkachsen mit dem tatsächlichen Schwerpunkt (SP) zur Deckung gebracht wird.

15. Stativ nach einem der vorhergehenden Ansprüche 1-10, **dadurch gekennzeichnet, dass** das Informationssystem einen Signal-Sender (72) umfasst, der aus der Entfernung Steuersignale aus dem Rechner für Stellmotore an Signal-Empfänger (D1-D10) an den Zubehörteilen (102-104) sendet.

## Claims

1. Stand having an automatically regulated balancing system with electrically controlled balancing mechanisms and a computer (10, 18, 27, 48) together with a calculation program assigned to it for driving the balancing mechanisms, having at least one component (35) movably connected to the stand, and having exchangeable accessories (36, 37) that can be detachably connected to the component (35), **characterized in that** the computer (10, 18, 27, 48) is assigned an information system (46), and the accessories (36, 37) have at least one data medium (38, 39, 40, 41), which can be read out by the information system (46) by means of a reader (19), for balance correction data of the accessories (36, 37), or **in that** the accessories (36, 37) have at least one readable data medium (38, 39) for balance correction data for the accessories (36, 37) which can be read out by an operator, and of which the data can be input into the information system.

2. Stand according to Claim 1, **characterized in that** the computer (27) comprises a calculation program which - if appropriate, as a function of angular or displacement positions of parts of the accessory (36) or of accessory parts (37) which can move relative to one another - controls a relative movement between the pivot axes (XDA/YDA/ZDA) and the centroid axes (XSP/YSP/ZSP) of the relevant component such that the point of intersection (D) of the pivot axes (XDA/YDA/ZDA) lies at the overall centroid (SP) of the movable component.

3. Stand according to Claim 1 or 2, **characterized in that** the information system (46) has a data input unit for data contained on the data medium of the accessory (47).

4. Stand according to Claim 3, **characterized in that** the data input unit has at least one manually operable keyboard (51) and a display (52).

5. Stand according to Claim 3, **characterized in that** the data input unit comprises an automatic reader (19) for the data stored on the data medium (38, 29) of the accessories (36, 37).

6. Stand according to one of the preceding claims, **characterized in that** movable components, a displaceable or pivotable accessory (42) or accessory parts (43) which can be displaced or pivoted thereon have position- and/or angle-measuring systems which, in the event of changes in the setup, pass on to the computer (48) corresponding, additional correction data to be taken into account during the balancing operation.

7. Stand according to one of the preceding claims, **characterized in that** the information system comprises a signal receiver (72) which can pick up at a distance signals which are sent by signal transmitters (D1-D10) to the accessory parts (102-104).

8. Stand according to Claim 7, **characterized in that** the signal transmitters (D1-D10) are frequency- or colour-coded light emitting diodes, ultrasound transmitters or discriminating reflector elements which can be excited by extraneous light.

9. Stand according to one of the preceding claims, **characterized in that** the information system (46) comprises telecommunication devices such as, for example infrared, ultrasound or radio transceivers.

10. Stand according to one of the preceding claims, **characterized in that** the data medium (41) comprises at least one intelligent chip which in the operating state - if appropriate, via sensors (45) arranged on the accessory (47) - automatically determines balance-relevant data and supplies them to the information system (46).

11. Method for automatically balancing regulated balancing systems on a stand by means of a computer (48), having one or more components (22, 35) movably connected to the stand, and having an exchangeable accessories (36, 37, 47) which can be detachably connected to a fastening base, **characterized in that**
a) the weight and the centroid position of the mounted accessories (36, 37, 47) with reference to the fastening base (35) are acquired digitally by means of an information system (46), and passed on for correction to the computer (48), or **in that**
b) the weight and the centroid position of the accessories (36, 37, 47) at the fastening point on a data medium (37, 38, 40, 41, 44) are specified, and **in that** an operator inputs these data for balance correction of the stand into an information system (46),which is connected to the computer (48), and **in that** in both cases a) and b) the computer (48) subsequently directly drives positioning motors for balancing purposes.

12. Method according to Claim 11, **characterized in that** the respective displacement or angular positions of movable accessories (37, 38, 40, 41, 44) or movable accessory parts (43) are acquired, and are input into the computer (48) automatically via the information system (46) for the purpose of additional correction of the balancing operation.

13. Method according to Claim 12, **characterized in that** the accessories is assigned a chip (33) with dedicated logic which automatically determines the displacement and angular positions, and of which parameters of program relevance for the computer are determined and are input directly into the computer (48) via the information system (46) such that the latter need not carry out any further additional calculations with reference to such unbalance states produced by changes in position or angle, but accepts as standard data all the data supplied by the information system (46).

14. Method according to one of Claims 11 to 13, **characterized in that** a local centroid coordinate system is defined, and the actual centroid (SP) of the movable component (35) is calculated in this coordinate system and the relative position or at least two pivot axes of the movable component (35) are determined by computation and the electric positioning motors (2, 3, 4) are driven such that the point of intersection (D) of these at least two pivot axes coincides with the actual centroid (SP).

15. Stand according to one of the preceding Claims 1-10, **characterized in that** the information system comprises a signal transmitter (72) which remotely transmits control signals from the computer for positioning motors to signal receivers (D1-D10) at the accessory parts (102-104).

## Revendications

1. Support comprenant un système d'équilibrage régulable automatiquement avec des mécanismes d'équilibrage à commande électrique et un ordinateur (10, 18, 27, 48) avec un programme informatique associé pour la commande des mécanismes d'équilibrage, avec au moins un composant (35) associé de manière mobile au support et un accessoire (36, 37) remplaçable pouvant être connecté de manière détachable au composant (35), **caractérisé en ce qu'**un système d'information (46) est associé à l'ordinateur (10, 18, 27, 48) et l'accessoire (36, 37) présente au moins un support de données (38, 39, 40, 41) pour des données de correction d'équilibrage de l'accessoire (36, 37), qui peuvent être lues par le système d'information (46) au moyen d'un appareil de lecture (19), ou **en ce que** l'accessoire (36, 37) présente au moins un support de données lisible (38, 39) pour des données de correction d'équilibrage de l'accessoire (36, 37), qui peut être lu par un opérateur et dont les données peuvent être saisies dans le système d'information.

2. Support selon la revendication 1, **caractérisé en ce que** l'ordinateur (27) comprend un programme informatique qui, éventuellement en fonction de positions angulaires ou de positions en translation de pièces mobiles les unes par rapport aux autres de l'accessoire (36) ou de pièces accessoires (37) - commande un mouvement relatif entre les axes de pivotement (XDA/YDA/ZDA) et les axes des centres de gravité (XSP/YSP/ZSP) du composant concerné, de telle sorte que le point d'intersection (D) des axes de pivotement (XDA/YDA/ZDA) se trouve au centre de gravité d'ensemble (SP) du composant mobile.

3. Support selon la revendication 1 ou 2, **caractérisé en ce que** le système d'information (46) présente une unité de saisie de données pour les données contenues sur le support de données de l'accessoire (47).

4. Support selon la revendication 3, **caractérisé en ce que** l'unité de saisie des données présente au moins un clavier actionnable manuellement (51) et un affichage (52).

5. Support selon la revendication 3, **caractérisé en ce que** l'unité de saisie des données comprend un appareil de lecture automatique (19) pour les données mémorisées sur le support de données (38, 29) de la pièce accessoire (36, 37).

6. Support selon l'une quelconque des revendications précédentes, **caractérisé en ce que** des composants mobiles, l'accessoire déplaçable en translation ou pivotant (42) ou des pièces accessoires (43) déplaçables en translation ou pivotant sur celui-ci présentent des systèmes de mesure de distance et/ou d'angle, qui transfèrent à l'ordinateur (48), en cas de changements de position, des données de correction correspondantes supplémentaires dont il faut tenir compte lors de l'opération d'équilibrage.

7. Support selon l'une quelconque des revendications précédentes, **caractérisé en ce que** le système d'information comprend un récepteur de signaux (72) qui peut recevoir des signaux éloignés, qui sont envoyés par des émetteurs de signaux (D1-D10) aux pièces accessoires (102-104).

8. Support selon la revendication 7, **caractérisé en ce que** les émetteurs de signaux (D1-D10) sont des diodes luminescentes à codage fréquence ou couleur, des émetteurs à ultrasons ou des éléments réflecteurs différentiables excitables par une source de lumière extérieure.

9. Support selon l'une quelconque des revendications précédentes, **caractérisé en ce que** le système d'information (46) comprend des dispositifs de transmission à distance, comme par exemple des émetteurs-récepteurs à infrarouge, ultrasons ou radio.

10. Support selon l'une quelconque des revendications précédentes, **caractérisé en ce que** le support de données (41) comprend au moins une puce intelligente, qui, dans l'état de fonctionnement - éventuellement par le biais de capteurs (45) disposés sur l'accessoire (47) - détecte automatiquement des données pertinentes pour l'équilibrage et les fournit au système d'information (46).

11. Procédé pour l'équilibrage automatique de systèmes d'équilibrage régulables sur un support au moyen d'un ordinateur (48), avec un ou plusieurs composants (22, 35) connectés de manière mobile au support et par son intermédiaire à un accessoire remplaçable (36, 37, 47) pouvant être connecté de manière détachable à une base de fixation,
**caractérisé en ce que**
a) le poids et la position du centre de gravité de l'accessoire monté (36, 37, 47) par rapport à la base de fixation (35) sont détectés numériquement au moyen du système d'information (46) et transmis à l'ordinateur (48) en vue d'effectuer une correction, ou **en ce que**
b) le poids et la position du centre de gravité de l'accessoire (36, 37, 47) au point de fixation sont indiqués à un support de données (37, 38, 40, 41, 44) et **en ce qu'**un opérateur saisit ces données, en vue d'effectuer une correction de l'équilibre du support, dans un système d'information (46) qui est connecté à l'ordinateur (48), et **en ce que**
dans les deux cas a) et b), l'ordinateur (48) commande ensuite directement des moteurs de commande électrique pour effectuer l'équilibrage.

12. Procédé selon la revendication 11, **caractérisé en ce que** les positions en translation ou angulaires respectives de l'accessoire mobile (37, 38, 40, 41, 44) ou des pièces accessoires mobiles (43) sont détectées et saisies dans l'ordinateur (48) par le biais du système d'information (46) automatiquement pour effectuer une correction ultérieure de l'opération d'équilibrage.

13. Procédé selon la revendication 12, **caractérisé en ce que** l'on associe à l'accessoire une puce (33) avec une logique qui détecte automatiquement les positions en translation et angulaires et leur paramètres pertinents du point de vue du programme pour l'ordinateur, et les injecte par le biais du système d'information (46) directement dans l'ordinateur (48), de sorte que celui-ci ne doive effectuer aucun autre calcul supplémentaire par rapport à des états de déséquilibre provoqués par de telles modifications de position ou angulaires, mais reprenne toutes les données fournies par le système d'information (46) comme données normalisées.

14. Procédé selon l'une quelconque des revendications 11 à 13, **caractérisé en ce qu'**un système de coordonnées du centre de gravité est défini et le centre de gravité proprement dit (SP) du composant mobile (35) est calculé dans ce système de coordonnées et la position relative d'au moins deux axes de pivotement du composant mobile (35) est déterminée par calcul, et les moteurs de commande électrique (2, 3, 4) sont commandés de telle sorte que le point d'intersection (D) de ces aux moins deux axes de pivotement soit amené en coïncidence avec le centre de gravité proprement dit (SP).

15. Support selon l'une quelconque des revendications précédentes 1 à 10, **caractérisé en ce que** le système d'information comprend un émetteur de signaux (72) qui envoie à distance des signaux de commande de l'ordinateur pour des moteurs de commande à des récepteurs de signaux (D1-D10) sur les pièces accessoires (102-104).
